Europäisches Patentamt

(19) European Patent Office ·

Office européen des brevets

(11) Veröffentlichungsnummer: **0 131 114**
B1

# (12) EUROPÄISCHE PATENTSCHRIFT

(45) Veröffentlichungstag der Patentschrift:
26.10.88

(21) Anmeldenummer: **84105479.4**

(22) Anmeldetag: **15.05.84**

(51) Int. Cl.⁴: **C 12 N 1/04,** A 21 D 8/04,
A 23 C 9/123, A 23 K 1/00

(54) Verfahren zum Herstellen eines trockenen lagerstabilen Bakterienpräparates.

(30) Priorität: **08.07.83 DE 3324644**

(43) Veröffentlichungstag der Anmeldung:
**16.01.85 Patentblatt 85/3**

(45) Bekanntmachung des Hinweises auf die Patenterteilung:
**26.10.88 Patentblatt 88/43**

(84) Benannte Vertragsstaaten:
**AT DE FR GB NL**

(56) Entgegenhaltungen:
**EP - A - 0 006 671
EP - A - 0 063 438
AT - B - 360 460
DE - A - 2 813 714**

**FOOD SERVICE TECHNOLOGY ABSTRACT, no.
81-07-P1141 (810338296); D.N. GANDHI: "Spray-dried
acidophilus milk powder" & INDIAN DAIRYMAN Band
32, Nr. 8, Seiten 599-600, 1980**

(73) Patentinhaber: **HÜLS AKTIENGESELLSCHAFT,
Patentabteilung / PB 15 - Postfach 13 20,
D-4370 Marl 1 (DE)**

(72) Erfinder: **Hill, Frank Friedrich, Dr., Schlesienstrasse 23,
D-4020 Mettmann (DE)**

## Beschreibung

Die Erfindung betrifft ein Verfahren zum Herstellen eines trockenen lagerstabilen pulver- oder granulatförmigen Präparates mit revitalisierbaren Bakterien, das nach dem Rehydratisieren mindestens $10^9$ lebensfähige Zellen pro Gramm des Präparates enthält und das frei von physiologisch oder pharmakologisch bedenklichen Zusatzstoffen ist.

Derartige Bakterienpräparate können als Starterkulturen vorteilhaft verwendet werden bei der Herstellung von Nahrungsmitteln (wie Backwaren aus Sauerteig und gesäuerte Milchprodukte) oder von gesäuerten Futtermitteln, weil sie sich mit den Stoffen, in die sie eingebracht werden sollen, leicht und homogen mischen lassen. Wegen der Lagerstabilität dieser Präparate wird die Herstellung solcher Nahrungsmittel vereinfacht; die Reinkulturen der Bakterien können weitgehend unabhängig von ihrer weiteren Verwendung gezüchtet werden.

Es ist bekannt, Bakterien durch Sprühtrocknen im Luftstrom in einen inaktiven Zustand zu versetzen, wobei ein pulverförmiges Produkt erhalten wird. Dabei werden jedoch die meisten Zellen getötet; der Anteil der lebensfähigen Zellen in diesen Produkten liegt oft unter 5% der ursprünglich vorhandenen lebenden Zellen, auch bei niedriger Lufttemperatur während der Trocknung. Weiter ist bekannt, eine Bakterienkultur durch schockartiges Einfrieren und anschliessendes Trocknen bei niedriger Temperatur unter Vakuum in ein pulverförmiges Produkt umzuwandeln, das überwiegend lebensfähige Zellen enthält. Dieses Pulver ist im tiefgefrorenen Zustand unter Luftabschluss einige Monate lagerfähig und mit gutem Erfolg revitalisierbar. Die Zellen lassen sich jedoch erst nach einer längeren Ruhepause wiederbeleben (R.K. Robinson in Biotechnology Vol. 3 (1983), Seite 197; Herausgeber H.W. Dellweg; Verlag Chemie).

In EP-0 063 438 ist ein Verfahren zur Herstellung eines trocknen Bakterienpräparates angegeben. Die Erzeugung spezieller Bakterienstämme, die weniger wärmeempfindlich sind, ist aufwendig; ferner ist es dabei schwierig, andere wünschenswerte Eigenschaften der Bakterien nicht zu beeinträchtigen. Beim Trocknen der Bakterien wird der grösste Teil inaktiviert, auch dann, wenn die Trocknungstemperatur unterhalb etwa 140 °C liegt. Das trockne Pulver enthält – je nach eingesetztem Bakterienstamm – $2 \cdot 10^5$ bis $5 \cdot 10^5$ lebensfähige Zellen pro Gramm Pulver. Die bei diesem Verfahren gewählten milden Trocknungsbedingungen, die zu dem «low-heat»-Produkt führen, können ungeeignete Bakterienstämme schädigen.

Bei der Herstellung von Backwaren mittels Sauerteig ist es oft notwendig, die Qualität eines biologischen Sauerteigs durch den gezielten Einsatz einer oder mehrerer Starterkulturen aus der Gruppe der Lactobacillen und/oder Propionibakterien gegenüber spontanen und unkontrollierten Gärungen zu verbessern. Es ist bekannt, handelsübliche Teigstücke, die durch Fermentation mit geeigneten Starterkulturen versäuert wurden, als Reinzuchtsauer zu vertreiben. Diese Produkte erfordern einen Frischdienst und eine kühle Lagerung, da sie nur eine begrenzte Haltbarkeit aufweisen. In der AT-PS 360 460 wird vorgeschlagen, die Lagerstabilität eines solchen fertigen Sauerteigs durch Granulieren und Trocknen im Wirbelbett zu verbessern. Die damit erhaltenen Produkte weisen jedoch nur einen geringen Gehalt an revitalisierbaren Lactobacillen auf, da die meisten Bakterien durch den Trocknungsprozess abgetötet werden.

Bisher ist kein technisch realisierbares Verfahren bekannt, mit dem Bakterienzellmasse ohne Zusatzstoffe in eine granulierte und wirbelfähige Form überführt werden kann, um diese im Wirbelbett schonend trocknen zu können.

Damit stellt sich die Aufgabe, Bakterienpräparate der obengenannten Art herzustellen, die in der Nahrungsmittelindustrie und in der Futtermittelherstellung verwendet werden können.

Die Aufgabe wird erfindungsgemäss gelöst durch ein Verfahren mit folgenden kennzeichnenden Merkmalen:
– Aufsprühen einer Zellsuspension des Stammes Lactobacillus brevis DSM 2647 oder einer Mischung dieses Stammes mit anderen Bakterienstämmen auf Getreideschrot und/oder Getreidemehl als Trägermasse, und
– Trocknen mittels heisser Luft bei einer Temperatur der besprühten Trägermasse von 20 bis 90 °C auf einen Gehalt von 60 bis 95 Massen-% Trockensubstanz,
wobei ein Bakterienpräparat entsteht, das wenigstens $10^9$ Zellen pro Gramm enthält, die nach dem Rehydratisieren lebensfähig sind, und das frei von physiologisch oder pharmakologisch bedenklichen Zusatzstoffen ist.

Das Bakterienpräparat wird unter Sauerstofffreiem Schutzgas verpackt.

Zum Herstellen eines Reinzuchtsauers eignet sich besonders eine Zellsuspension des Bakterienstammes Lactobacillus brevis DSM 2647, die im Wirbelbett auf Getreideschrot oder Getreidemehl als Trägersubstanz aufgesprüht und mittels heisser Luft bei Produkttemperaturen von 20 bis 40 °C getrocknet wird. Das fertige Präparat besteht aus 88 bis 94% Trockensubstanz und enthält mindestens $10^9$ und bis zu $10^{11}$ lebensfähige Bakterien pro Gramm. Die aufgesprühte Zellsuspension kann auch eine Mischung aus den säurebildenden Bakterien und anderen Mikroorganismenstämmen wie beispielsweise Backhefe enthalten. Dieser Reinzuchtsauer wird nach bekannten Methoden über einen Anstellsauer zum Herstellen von Backwaren mittels Sauerteig verwendet.

Ein erfindungsgemäss hergestelltes trockenes Bakterienpräparat kann weiter zum Säuern von Getreideschrotmischungen verwendet werden, die beispielsweise zum Füttern von Schweinen eingesetzt wird. Solche biologisch gesäuerten Futtermittel haben günstige Auswirkungen auf Futteraufnahme und Gesunderhaltung der Tiere.

Eine Starterkultur zum Herstellen von Milchprodukten wie Dickmilch und Sauerrahm erhält man durch Aufsprühen einer Zellsuspension von Lactobacillus casei DSM 2648 oder DSM 2649 auf Milchpulver als Trägermasse, die im Wirbelbett mittels heisser Luft bei Produkttemperaturen von 20 bis 40 °C getrocknet und anschliessend mit entfeuchteter Luft (Taupunkt der Luft unterhalb − 15 °C) nachgetrocknet wird. Ein solches Bakterienpräparat besteht aus 94% Trockensubstanz und enthält mindestens $10^9$ und bis zu $10^{11}$ lebensfähige Bakterien pro Gramm.

Die erfindungsgemässen Bakterienpräparate werden unter Sauerstoff-freiem Schutzgas abgepackt; sie sind bei Raumtemperatur mehrere Monate lagerfähig.

Das erfindungsgemässe Verfahren hat folgende Vorteile:

- Die Bakterien werden sehr schonend getrocknet und in den inaktiven Zustand überführt, wobei die meisten Zellen lebensfähig bleiben.
- Der Gehalt an lebensfähigen Zellen im Bakterienpräparat kann auf sehr grosse Werte gebracht werden.
- Die Bakterienpräparate fallen ohne zusätzliche Verfahrensschritte pulver- oder granulatförmig an und enthalten keine für ihre Verwendung artfremden Zusatzstoffe.
- Die Präparate sind unter Schutzgas bei Raumtemperatur lagerfähig.

Die erwähnten Bakterienstämme von Lactobacillus brevis und Lactobacillus casei (DSM 2647, DSM 2648 und DSM 2649) wurden aus Gras-Silage bzw. Milchprodukten isoliert und aufgrund ihrer Morphologie und Säurebildung identifiziert.

Lactobacillus brevis DSM 2647 erweist sich nach Anzucht in flüssigen Kulturmedien, Aufkonzentrierung durch Zentrifugieren, Ansprühen auf Mehl als Trägerstoff und anschliessendem Trocknen anderen Lactobacillen-Stämmen aus Mikroorganismen-Sammlungen als deutlich überlegen. Die Überlebensrate dieses Stammes liegt nach dem Trocknen auf 92% Trockensubstanz bei über 50%. Daneben bildet der Stamm in Mehlteigen aus Maltose rasch Milchsäure, Essigsäure und Kohlendioxid.

Die Erfindung wird durch folgende Beispiele erläutert, ohne hierauf beschränkt zu sein.

Beispiel 1
Trockener Reinzuchtsauer für Backwaren

Lactobacillus brevis DSM 2647 wird in einer Nährlösung aus

| | |
|---|---|
| 4% | Maltose-Sirup |
| 1% | geklärtem Maisquellwasser |
| 1% | Hefeextrakt |
| 0,5% | $CaCO_3$ |
| 0,06% | $MgSO_4 \cdot 7H_2O$ |
| 0,01% | $MnSO_4 \cdot H_2O$ |
| 0,0034% | $FeSO_4 \cdot 7H_2O$ |

bei pH 6,0 geimpft und bei schwacher Belüftung 24 Stunden bei 33 °C bebrütet. Die Bakterienzellzahl steigt von $10^6$ Zellen/ml auf $6 \cdot 10^9$ Zellen/ml an. Die Kulturbrühe wird bei 3000 rpm in einer Zentrifuge in Bakterienzellmasse und Überstand getrennt. Das Sediment wird in wenig Kulturbrühe suspendiert, dabei wird eine Zellzahl von $5 \cdot 10^{10}$ Zellen/ml erreicht. 400 ml dieser Zellsuspension werden auf 600 g Mehl in einem Wirbelbettapparat aufgesprüht. Das Mehl wird dabei in einem Luftstrom fluidisiert. Die Temperatur des Produktes wird durch Aufheizen des Luftstromes bei 30 °C gehalten. In 15 Minuten ist die Zellsuspension aufgetragen, anschliessend wird etwa 30 Minuten mit einem getrockneten und vorgewärmten Luftstrom (Taupunkt der Luft unterhalb − 15 °C) auf einen Gehalt von 91% Trockensubstanz getrocknet. Die Anzahl der lebensfähigen Keime wird nach dem Rehydratisieren und entsprechendem Verdünnen bestimmt. Der Gehalt an lebensfähigen Lactobacillen in sprühgetrocknetem Produkt beträgt $2,6 \cdot 10^{10}$ Zellen/g, d.h. mehr als 50% der Zellen haben die Trocknung lebend überstanden.

Der nach dem erfindungsgemässen Verfahren mit Lactobacillus brevis DSM 2647 hergestellte trockene und lagerstabile Reinzuchtsauer erreicht Keimzahlen von $10^9$ bis $10^{11}$ Keime pro g Produkt. Nach Abpacken unter Schutzgas wird innerhalb von 3 Monaten kein nennenswerter Abfall der Keimzahl bei Lagerung bei Raumtemperatur beobachtet.

Beispiel 2
Anstellsauer aus Reinzuchtsauer

Aus 1 g sprühgetrocknetem Reinzuchtsauer mit dem Stamm Lactobacillus brevis DSM 2647 wird ein Anstellsauer bereitet. Der Zelltiter des eingesetzten Produktes beträgt nach dem Rehydratisieren $2 \cdot 10^{10}$ Zellen/g Produkt. Als Vergleichsversuch wird ein Anstellsauer aus 1 g gefriergetrocknetem Lactobacillus brevis DSM 20 054 bereitet. Der Zelltiter dieses eingesetzten Produktes beträgt nach dem Rehydratisieren $6 \cdot 10^{10}$ Zellen/g Produkt.

Der Anstellsauer wird angesetzt mit 1 g Reinzuchtsauer, 280 g Roggenmehl, 5,6 g Kochsalz und 280 ml Wasser. Der Teig wird 16 Stunden bei 30 °C inkubiert. Nach dieser Standzeit wird ein Vollsauer angesetzt, der folgende Zusammensetzung hat: 28 g Anstellsauer, 280 g Roggenmehl, 5,6 g Kochsalz, 280 ml Wasser. Der Ansatz wird bei 30 °C inkubiert, und der Abfall des pH-Wertes wird gemessen. Wie Tabelle 1 zeigt, ist der aus dem sprühgetrockneten Reinzuchtsauer bereitete Ansatz dem Vergleichsansatz deutlich überlegen.

Tabelle 1
Säuerungsgeschwindigkeit der Anstellsauer beim Übergang in Vollsauer

| | pH-Wert nach | | | | | |
|---|---|---|---|---|---|---|
| | 0 h | 2 h | 4 h | 6 h | 8 h | 10 h |
| erfindungsgemäss hergestellter Reinzuchtsauer mit Lactobacillus brevis DSM 2647 | 5,6 | 5,5 | 5,1 | 4,6 | 4,2 | 3,8 |
| gefriergetrockneter Reinzuchtsauer mit Lactobacillus brevis DSM 20 054 | 5,7 | 5,7 | 5,6 | 5,4 | 5,1 | 4,8 |

Wie die Abnahme des pH-Wertes zeigt, hat der Vollsauer aus erfindungsgemäss hergestelltem Reinzuchtsauer seine Reife bereits nach 8 h erreicht, während der Vollsauer aus gefriergetrocknetem Reinzuchtsauer nach 10 h noch nicht genügend gesäuert ist.

Beispiel 3
Lagerstabilität des erfindungsgemäss hergestellten Bakterienpräparates (Reinzuchtsauer) mit Lactobacillus brevis DSM 2647

Jeweils 5 g dieses erfindungsgemäss hergestellten Bakterienpräparates werden in Ampullen gefüllt, evakuiert und mit Stickstoff gespült und abgeschmolzen. Die Behältnisse werden bei 35°C gelagert. Nach verschiedenen Zeiten wird jeweils eine Ampulle geöffnet, und das Präparat wird rehydratisiert. Der Titer an lebenden Zellen pro g Präparat wird durch Ausplattieren auf geeigneten Nährboden bestimmt.

Wie Tabelle 2 zeigt, ändert sich die Anzahl der lebenden Zellen während der Lagerzeit von 12 Wochen im Rahmen der Zählgenauigkeit praktisch nicht; damit ist das erfindungsgemäss hergestellte Bakterienpräparat unter den angegebenen Bedingungen, die einer Verpackung in technischem Massstab (im Mehrschichten-Verbundfolienbeutel mit Schutzbegasung) entsprechen, ausreichend lagerfähig.

Tabelle 2
Abnahme der Anzahl lebender Zellen während der Lagerung

| Lagerzeit Wochen | Anzahl lebender Zellen Zellen/g Bakterienpräparat |
|---|---|
| 0 | $2,5 \cdot 10^{10}$ |
| 3 | $2,3 \cdot 10^{10}$ |
| 6 | $1,6 \cdot 10^{10}$ |
| 9 | $1,8 \cdot 10^{10}$ |
| 12 | $2,3 \cdot 10^{10}$ |

Beispiel 4
Bereitung von Roggenmischbrot mittels erfindungsgemäss hergestelltem Bakterienpräparat

Es wird die Berliner Kurzsauerführung angewendet, die mit 3 bis 4 Stunden Reifezeit eine sehr kurze Führung ist. Als Mehlmischung wird 70% Roggenmehl Type 1150 und 30% Weizenmehl Type 1050 verwendet. Der Saueranteil beträgt 40% bezogen auf die gesamte Mehlmenge. Der Kurzsauer wird hergestellt aus 2 kg Mehlmischung, 0,4 kg Anstellgut (hergestellt gemäss Beispiel 2 als Vollsauer) und 1,8 kg Wasser. Bei einer Teigausbeute von 190 wird eine Reifezeit von 3,5 Stunden bei 35°C eingehalten.

Der Teig wird zubereitet aus
3,8 kg Kurzsauer
1,5 kg Roggenmehl
1,5 kg Weizenmehl
0,1 kg Hefe
0,1 kg Salz
1,6 kg Wasser

Bei einer Teigausbeute von 168 wird bei einer Teigtemperatur von 28°C und einer Teigruhezeit von 10 min auf Stückgare gestellt. Nach dem Abbacken erhält man ein einwandfreies Roggenmischbrot, das sich durch einen abgerundeten milden Geschmack und ein gleichmässiges Porenbild auszeichnet.

Beispiel 5
Trockenes Bakterienpräparat als Starterkultur für die Quark-Herstellung

Lactobacillus casei DSM 2648 wird in einer Nährlösung aus

| | |
|---|---|
| 1% | geklärtem Maisquellwasser |
| 1% | Hefe-Extrakt |
| 3% | Magermilchpulver |
| 0,06% | $MgSO_4 \cdot 7H_2O$ |
| 0,0034% | $FeSO_4 \cdot 7H_2O$ |
| 0,01% | $MnSO_4 \cdot H_2O$ |

bei pH 6,0 während 20 Stunden bei 30°C inkubiert. Der pH-Wert des Mediums fällt in dieser Zeit von 6,0 auf 3,6; der Zellgehalt steigt auf $5,1 \cdot 10^9$ Zellen pro Milliliter an.

Die Kulturbrühe wird – wie in Beispiel 1 beschrieben – von der Zellmasse abgetrennt. Die Zellmasse wird in einer zehnprozentigen Magermilch-Suspension aufgeschwemmt; der Zellgehalt steigt auf $7 \cdot 10^{10}$ Zellen/ml.

500 ml dieser Suspension werden auf 1000 g Magermilchpulver unter den in Beispiel 1 beschriebenen Bedingungen aufgesprüht und im Wirbelbett getrocknet. Das trockene Bakterienpräparat enthält $3 \cdot 10^{10}$ lebensfähige Zellen pro Gramm.

Pasteurisierte Rohmilch wird durch 0,1% Inoculum des trockenen Bakterienpräparates in weniger als 24 Stunden gesäuert, wobei das Casein als feste Quarkmasse koaguliert wird.

Beispiel 6
Versäuerung von Getreideschrot für die Schweinefütterung

1 Gewichtsteil geschrotete Gerste, 0,25 Teile Sojaschrot und 1,5 Teile Wasser werden zu einem Brei verrührt und 24 Stunden bei Raumtemperatur stehengelassen. Ein aliquoter Teil des Breies wird mit 0,25% sprühgetrocknetem Bakterienpräparat (Lactobacillus brevis DSM 2647 auf Mehl als Träger), das entsprechend Beispiel 1 hergestellt wird, versetzt; dieser Brei wird ebenfalls 24 Stunden bei Raumtemperatur stehengelassen.

Die saprophytische mikrobielle Flora in beiden Ansätzen wird durch Plattieren einer geeigneten Verdünnung des Futterbreies auf zwei Nährböden mit folgender Zusammensetzung bestimmt:

| Nährboden A | Nährboden B |
|---|---|
| 2% Hefe-Extrakt | 2% Hefe-Extrakt |
| 1% Pepton | 1% Pepton |
| 2% Glycerin | 2% Glucose |
| 2% Agar | 2% Agar |
| pH = 6,5 | pH = 6,5 |

Die Platten werden 3 Tage bei 30 °C bebrütet, und zwar die Platten des Nährbodens A unter aeroben Bedingungen, die Platten des Nährbodens B unter anaeroben Bedingungen.

Die saprophytische Flora des Futterbreies wird in dem mit dem Bakterienpräparat versetzten Ansatz, dessen pH-Wert von 6,2 auf 4,5 nach dem Stehenlassen gesunken ist, in günstiger Weise beeinflusst.

Während die Anzahl der unerwünschten und möglicherweise pathogen wirkenden Saprophyten im Futterbrei ohne Bakterienzusatz nach 24 Stunden Standzeit deutlich zunimmt, nimmt diese Anzahl in dem mit dem Bakterienpräparat versetzten Futterbrei merklich ab. Gleichzeitig erreicht die Anzahl der erwünschten Lactobacillen im Futterbrei ohne Bakterienzusatz nur unbedeutende Werte, während in dem mit dem Bakterienpräparat versetzten Futterbrei diese Anzahl kräftig zunimmt (siehe Tabelle 3). Dadurch wird ein positiver Effekt auf die Gesunderhaltung und auf die Futteraufnahme der Schweine, die mit gesäuertem Futterbrei gefüttert werden, erreicht. Das gesäuerte Futter wird von Schweinen gern aufgenommen.

Tabelle 3

Veränderung der mikrobiellen Flora im Futterbrei mit und ohne Zusatz des sprühgetrockneten Bakterienpräparats

| | mit dem Bakterienpräparat versetzter Futterbrei Keime pro Gramm | Futterbrei ohne Zusatz Keime pro Gramm |
|---|---|---|
| Anzahl der unerwünschten Saprophyten nach einer Standzeit von | | |
| 0 Stunden | $1,3 \cdot 10^6$ | $1,3 \cdot 10^6$ |
| 24 Stunden | $2,4 \cdot 10^4$ | $8,5 \cdot 10^7$ |
| Anzahl der Lactobacillen nach einer Standzeit von | | |
| 0 Stunden | $2 \cdot 10^7$ | $< 10^5$ |
| 24 Stunden | $8 \cdot 10^8$ | $2 \cdot 10^7$ |

## Patentansprüche

1. Verfahren zum Herstellen eines trockenen, lagerstabilen pulver- oder granulatförmigen Präparates mit revitalisierbaren Bakterien durch Aufsprühen einer Zellsuspension aus mindestens einem Bakterienstamm auf eine im Wirbelbett fluidisierte pulver- oder granulatförmige Trägermasse, Trocknen der besprühten Trägermasse im Wirbelbett mittels eines heissen Gases und Verpacken des Bakterienpräparates unter Sauerstofffreiem Schutzgas, gekennzeichnet durch
- Aufsprühen einer Zellsuspension des Stammes Lactobacillus brevis DSM 2647 oder einer Mischung dieses Stammes mit anderen Bakterienstämmen auf Getreideschrot und/oder Getreidemehl als Trägermasse, und
- Trocknen mittels heisser Luft bei einer Temperatur der besprühten Trägermasse von 20 bis 90 °C auf einen Gehalt von 60 bis 95 Massen-% Trockensubstanz,
wobei ein Bakterienpräparat entsteht, das wenigstens $10^9$ Zellen pro Gramm enthält, die nach dem Rehydratisieren lebensfähig sind, und das frei von physiologisch oder pharmakologisch bedenklichen Zusatzstoffen ist.

2. Verfahren nach Anspruch 1, gekennzeichnet durch
- Aufsprühen einer Zellsuspension der Stämme Lactobacillus casei DSM 2648 oder Lactobacillus casei DSM 2649 auf Milchpulver als Trägermasse.

3. Verfahren nach den Ansprüchen 1 und 2, gekennzeichnet durch
- Trocknen mittels heisser Luft bei einer Temperatur der besprühten Trägermasse von 20 bis 40 °C auf einen Gehalt von 85 bis 95 Massen-% Trockensubstanz.

4. Verwenden des nach Anspruch 1 hergestellten trockenen Bakterienpräparates als Reinzuchtsauer für die Herstellung von Backwaren mittels Sauerteig.

5. Verwenden des nach Anspruch 2 hergestellten trockenen Bakterienpräparates als Starterkultur für die Herstellung von gesäuerten Milchprodukten.

6. Verwenden des nach Anspruch 1 hergestellten trockenen Bakterienpräparates als Starterkultur für die Herstellung von gesäuerten Futtermitteln.

## Claims

1. A process for producing a dry, storage-stable preparation, containing revitalizable bacteria, in the form of a powder or granules by spraying a cell suspension of at least one strain of bacteria onto an excipient composition, in the form of a powder or granules suspended as a fluidized bed, drying the sprayed excipient composition in the fluidized bed by means of a hot gas, and packaging the preparation of bacteria under an oxygen-free protective gas, characterized in that
- a cell suspension of the strain Lactobacillus brevis DSM 2647 or a mixture of this strain with strains of other bacteria is sprayed onto coarse cereal meal and/or cereal flour as the excipient composition, and
- the sprayed excipient composition is dried at a temperature of 20 to 90 °C by means of hot air to a content of 60 to 95% by weight of dry matter, resulting in a preparation of bacteria containing at least 10⁹ cells per gram which are viable after rehydration, and which is free of physiologically or pharmacologically unacceptable additives.

2. A process according to claim 1, characterized in that
- a cell suspension of the strains Lactobacillus casei DSM 2648 or Lactobacillus casei DSM 2649 is sprayed onto milk powder as the excipient composition.

3. A process according to claim 1 or 2, characterized in that
- the sprayed excipient composition is dried at a temperature of 20 to 40 °C, by means of hot air, to a content of 85 to 95% by weight of dry matter.

4. The use of the dry preparation of bacteria produced in accordance with claim 1 as a purebreed sour dough for the production of baked goods by means of sour dough.

5. The use of the dry preparation of bacteria produced in accordance with claim 2 as a starter culture for the production of fermented milk products.

6. The use of the dry preparation of bacteria produced in accordance with claim 1 as a starter culture for the production of fermented animal feeds.

## Revendications

1. Procédé de fabrication d'une préparation sous forme de poudre ou de granulés, sèche et stable au stockage, avec des bactéries revitalisables, par pulvérisation d'une suspension de cellules provenant d'au moins une souche de bactéries sur une masse de support pulvérulente ou granulée fluidisée en lit tourbillonnant, séchage de la masse de support pulvérisée dans le lit fluidisé au moyen d'un gaz chaud et emballage de la préparation bactérienne sous gaz protecteur exempt d'oxygène, caractérisé par
- vaporisation d'une suspension de cellules de la souche Lactobacillus brevis DSM 2647 ou d'un mélange de cette souche avec d'autres souches bactériennes sur du gruau de céréales et/ou de la farine de céréales comme masse de support, et
- séchage au moyen d'air chaud à une température de la masse de support pulvérisée de 20 à 90 °C, jusqu'à une teneur de 60 à 95% en masse de substance sèche, ce qui donne naissance à une préparation bactérienne contenant au moins 10⁹ cellules par gramme, qui sont viables après la réhydratation, et qui est exempte d'additifs douteux du point de vue physiologique ou pharmaceutique.

2. Procédé selon la revendication 1, caractérisé par pulvérisation d'une suspension de cellules de la souche Lactobacillus casei DSM 2648 ou Lactobacillus casei DSM 2649 sur de la poudre de lait comme masse de support.

3. Procédé selon les revendications 1 et 2, caractérisé par séchage à l'aide d'air chaud à une température de la masse de support pulvérisée de 20 à 40 °C, jusqu'à une teneur de 85 à 95% en masse de substance sèche.

4. L'utilisation de la préparation bactérienne sèche préparée selon la revendication 1 en tant que culture acide pure pour la production de pain et pâtisserie au moyen de levain.

5. L'utilisation de la préparation bactérienne sèche préparée selon la revendication 2 en tant que culture de départ pour la production de laitages acidifiés.

6. L'utilisation de la préparation bactérienne sèche préparée selon la revendication 1 en tant que culture de départ pour la production de fourrages acidifiés.